# EUROPEAN PATENT SPECIFICATION

(11) **EP 2 682 461 B1**
(45) Date of publication and mention of the grant of the patent: **13.04.2016**
(21) Application number: 13165345.3
(22) Date of filing: 25.04.2013
(51) Int. Cl.: C12N 11/14, B09C 1/00

(54) **Carrier for immobilizing methanotroph and method for removing methane employing the same**
Träger zur Immobilisierung von Methanotroph und Verfahren zum Entfernen von Methan, das diesen einsetzt
Support pour immobiliser du méthanotrophe et procédé pour l'élimination de méthane utilisant celui-ci

(30) Priority: 25.04.2012 KR 20120043467
(43) Date of publication of application: 08.01.2014
(73) Proprietor: Ewha University-Industry Collaboration Foundation, Seoul 120-750 (KR)
(72) Inventor: Cho, Kyung Suk, 120-750 Seoul (KR); Kim, Tae Gwan, 120-750 Seoul (KR); Kim, Min Kyung, 120-750 Seoul (KR); Moon, Kyung Eun, 120-750 Seoul (KR)
(74) Representative: Regimbeau

(56) References cited:
- WO-A1-2011/007907
- J. STREESE ET AL: "Microbial oxidation of methane from old landfills in biofilters", WASTE MANAGEMENT, vol. 23, no. 7, 1 January 2003 (2003-01-01), pages 573-580, XP055088048, ISSN: 0956-053X, DOI: 10.1016/S0956-053X(03)00097-7
- JOSIANE NIKIEMA ET AL: "The Use of Inorganic Packing Materials during Methane Biofiltration", INTERNATIONAL JOURNAL OF CHEMICAL ENGINEERING, vol. 39, no. 3, 1 January 2010 (2010-01-01), pages 400-8, XP055088302, ISSN: 1687-806X, DOI: 10.1016/S1164-5563(01)01067-6
- MALGORZATA PAWOWSKA ET AL: "The effect of bed properties on methane removal in an aerated biofilter Model studies", WASTE MANAGEMENT, ELSEVIER, NEW YORK, NY, US, vol. 31, no. 5, 11 October 2010 (2010-10-11), pages 903-913, XP028164612, ISSN: 0956-053X, DOI: 10.1016/J.WASMAN.2010.10.005 [retrieved on 2010-10-18]
- HAUBRICHS R ET AL: "Evaluation of aerated biofilter systems for microbial methane oxidation of poor landfill gas", WASTE MANAGEMENT, ELSEVIER, NEW YORK, NY, US, vol. 26, no. 4, 1 January 2006 (2006-01-01), pages 408-416, XP024961187, ISSN: 0956-053X, DOI: 10.1016/J.WASMAN.2005.11.008 [retrieved on 2006-01-01]
- YUN JEONGHEE ET AL: "Comparison of Methanotrophic Activity at Top and Bottom Layers in Up-flow Biofilters", KOREAN JOURNAL OF MICROBIOLOGY AND BIOTECHNOLOGY, vol. 41, no. 2, June 2013 (2013-06), pages 221-227, XP002716395, ISSN: 1598-642X
- SO-YEON JEONG ET AL: "Effect of Tobermolite, Perlite and Polyurethane Packing Materials on Methanotrophic Activity", KOREAN JOURNAL OF MICROBIOLOGY AND BIOTECHNOLOGY, vol. 41, no. 2, 28 June 2013 (2013-06-28), pages 215-220, XP55087991, ISSN: 1598-642X, DOI: 10.4014/kjmb.1301.01005
- DATABASE WPI Week 200409 Thomson Scientific, London, GB; AN 2004-084972 XP002716396, & JP 2003 259867 A (ISHIKAWAJIMA HARIMA HEAVY IND) 16 September 2003 (2003-09-16)

<110> Ewha University-Industry Collaboration Foundation
<120> Carrier for immobilizing Methanotroph and method for removing methane employing the same
<130> OPA13041/EP
<150> KR 10-2012-0043467
   <151> 2012-04-25
<160> 4
<170> Kopatent In 2.0
<210> 1
   <211> 18
   <212> DNA
   <213> Artificial Sequence
<220>
   <223> 340F primer
<400> 1
   tcctacggga ggcagcag 18
<210> 2
   <211> 21
   <212> DNA
   <213> Artificial Sequence
<220>
   <223> 805R primer
<400> 2
   gactachvgg gtatctaatc c 21
<210> 3
   <211> 58
   <212> DNA
   <213> Artificial Sequence
<220>
   <223> forward primer
<400> 3
   ccatctcatc cctgcgtgtc tccgactcag nnnnnnnnnn tcctacggga ggcagcag 58
<210> 4
   <211> 51
   <212> DNA
   <213> Artificial Sequence
<220>
   <223> reverse primer
<400> 4
   cctatcccct gtgtgccttg gcagtctcag gactachvgg gtatctaatc c 51

## Description

### [Technical Field]

This invention relates to a carrier for use in removing methane comprising tobermolite-immobilized Methanotroph . In more detail, this invention relates to a carrier for use in removing methane comprising tobermolite-immobilized Methanotroph that can selectively allow Methanotroph to grow with high reproduction rate, biofilter that comprises immobilized Methanotroph in the carrier, biocover that comprises Methanotroph immobilized in the carrier, method for removing methane using the biofilter or biocover, use of a culture media comprising tobermolite for culturing selectively Methanotroph that can grow with high reproduction rate, and method for selectively culturing Methanotroph using the culture media.

### [Background Art]

Since the industrial revolution, phenomenon such as urbanization, industrialization, and population increase has caused various environmental issues. Among the issues, climate change caused by greenhouse gases has been recognized as a global environmental problem that is threatening earthious environmental issues. Among the issue, since United Nations Framework Convention on Climate Change (1992), imposition of international joint measure and various policy such as emission trading system and carbon tax are being established and implemented, while researches related to reduction of greenhouse gases are being actively conducted. Also, reduction in emission of greenhouse gases is notbeing expanded beyond industrial sector to sector of agricultureandstockbreeding as well as waste disposal.

Second main reason following carbon dioxide, Methane is one of the greenhouse gases that causes global climate change and the greenhouse effect caused by methane is 20 times stronger than those caused by carbon dioxide. One of main synthetic source factor of methane is landfill. It is estimated thatannual amount of methane caused by landfill is 35 to 73 Tg(tera gram). For this reason, there are active research activities to recover and use methane generated from landfills as energy source. However, for small scale landfill that would be economically inefficient to install a methane collection facility, or a landfill that has reached full capacity considerable time ago and discharges methane that is too low in concentration to be used as energy, there is no prepared measure to address this issue.

Some methods are known to be used for methane degradation. As example, using a mixture of compost, peat, and wood fiber was used as biofilter and showed stable and satisfactory rate of methane degradation around 20 g.m⁻³.h⁻¹ (Waste Management 23 (2003) 573-580). Another example was using inorganic packing materials as methane biofilter. This study showed that rock material having an average particule size of 2mm appeared to be efficient, which elimination capacity of methane was up to 50 g.m⁻³.h⁻¹ (International Journal of Chemical Engineering, Volume 2010, Art.ID 573149). Haubrichs et al. (Waste Management 26 (2006) 4008-416) and Pawlawska et al. (Waste Management 31 (2011) 903-913) shown that organic biofilters aerated were be used for methane oxidation. Using oxygen as aerator increase methane oxidation rate by a factor of 5.5 compared to the same test realized without oxygen. Microorganism support comprising cement type calcium-silicate increases also the efficiency of methane fermentation process (Japan Patent Publication No. JP2003259867).

In order to reduce amount of methane caused by landfill that outputs methane unusable for energy, researches relating to use of Methanotroph that consumes methane to proliferate, is being conducted. Since Methanotroph uses methane as carbon source and energy source to proliferate, using Methanotroph is known as an effective method for removing methane.

However, using the Methanotroph to remove methane caused by landfill carries a flaw in that the methane removal efficiency is very low.

Despite Methanotroph must be immobilized by inoculating to a mineral carrier such as illite and montmorillonite in order for Methanotroph to be used in a landfill, Methanotroph cannot normally proliferate or grow when inoculated to the mineral carrier, thereby resulting in low methane reduction efficiency even in case of usage of immobilized Methanotroph.

In order to resolve this issue, active research is being conducted to develop Methanotroph that shows excellent efficiency in methane removal. (Korean Patent Publication No. 2011-0006962; Korean Patent Publication No. 2011-0006964). Further, even if superior Methanorophs for removing methane are used, issue concerning the strain not being able to beeffectively immobilized for normal functioning still needs to be resolved.

Under these circumstances, these inventors discovered that selective and high proliferation of Methanotroph is feasible when using tobermolite, which is a type of mineral carrier, as an immobilized carrier for Methanotroph, thereby completing this invention.

### [Disclosure]

### [Technical Problem]

One of the objects of this invention is to provide a carrier for use in removing methane comprising tobermolite-immobilized Methanotroph.

Another object of this invention is to provide a biofilter that comprises tobermolite-immobilized Methanotroph carrier.

Another object of this invention is to provide a biocover comprising a biofilter that comprises tobermolite-immobilized Methanotroph carrier.

Another object of this invention is to provide a method to remove methane using the biofilter or biocover.

Another object of this invention is to provide a using of a culture media that comprises tobermolite for culturing selectively Methanotroph.

Another object of this invention is to provide a method to culture selectively Methanotroph using the media.

### [Technical Solution]

As an embodiment for achieving the object, this invention provides a carrier for use in removing methane comprising tobermolite-immobilized Methanotroph.

The terminology of this invention, "tobermolite", is a stabilized calcium silicate hydrate (5CaO. 6SiO₂. 5H₂O) that has high solidity which is produced from silica (SiO₂) and calcium through hydrothermal reaction. Tobermolite has porosous, light weight, solid and of crystal habit features, and is most effectively created when CaO/SiO₂ molar ratio is between from 0.5 to 1.5.

For purpose of this invention, the tobermolite can be used as a carrier for immobilizing Methanoroph as well as a culture media, although its usage is not limited to the described thereof.

The terminology of this invention, "Methanotroph", refers to an organism in the methylotroph category that uses only methane as a carbon source and energy source. Methanotroph absorbs methane at the exterior of a cell and transforms the absorbed methane to consecutive order of CH₃OH, HCHO, HCOOH and CO₂, thereby obtaining ATP by totally oxidizing methane to carbon dioxide. Methanotroph of this invention may be microorganism such as *Methylocystis* sp., *Methylosarcina* sp., and *Methylocaldum* sp., although not limited to the described thereof, as long as it can be immobilized to the tobermolite and have ability of eliminating methane.

The terminology of this invention, "methylotroph", refers to an organism that uses compounds with one carbon such as methane, methanol and methylamine, or compounds that did not C-C combine within a molecule such as dimethylamine and trimethylamine as carbon source and energy source. Methylotroph is categorized to either obligate methylotroph (*Methylococcus* sp., *Methylosinus* sp. etc.) that can only use the compounds, or faculative methylotroph (*Pseudomonas* sp., *Methylobacterium* sp. etc.) that can also use other carbon compounds. Methanotroph of this invention is categorized as obligate methylotroph that can use methane as only carbon source and energy source, although not limited to the described thereof.

The terminology of this invention, "carrier forimmobilizing" refers to substrate that immobilizes microorganism for industrial usage. The substrate has durability that is preferable for industrial usage as it can immobilize the target microorganism with high concentration, comprises multiple pore, can form reticulated network of flow channels that are formed by extension of the pore, as well as has sufficient mechanical strength such as elasticity and compressive strength.

For purpose of this invention, the carrier can be a carrier that is not inoculated with Methanotroph, a carrier that is already inoculated with Methanotroph, or a carrier inoculated with proliferated Methanotroph, although not limited to the described thereof.

Also, tobermolite that can immobilize and selectively allow Methanotroph to proliferate can be used alone, as well as combination of tobermolite and other mineral substances that can help proliferation of the Methanotroph can be used, although the above carrier is not limited by this. In case a combination of the tobermolite and other material substance is used as the carrier, the content of the tobermolite that is comprised in the carrier is preferably 10 to 90 wt %, more preferably 50 to 90 wt % and most preferably 80 to 90 wt % with reference to the total weight of the carrier, although not specifically limited to the description thereof if it may constitute the carrier that can be used for immobilizing and culturing of Methanotroph.

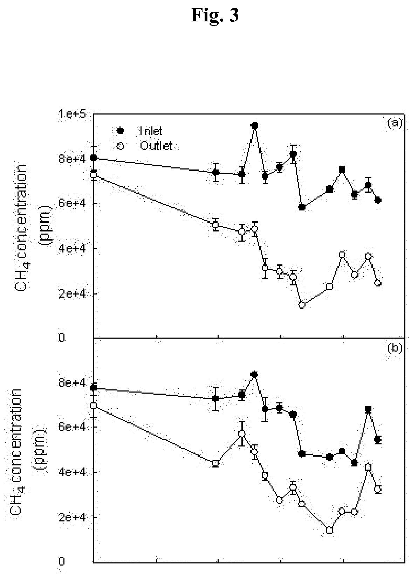

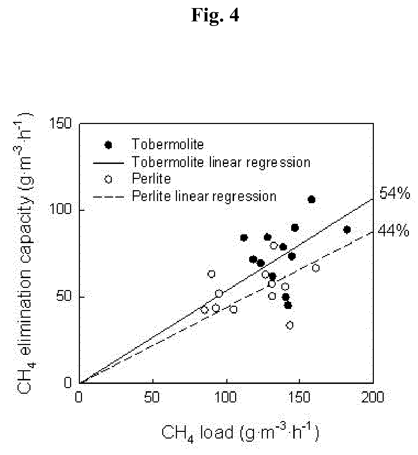

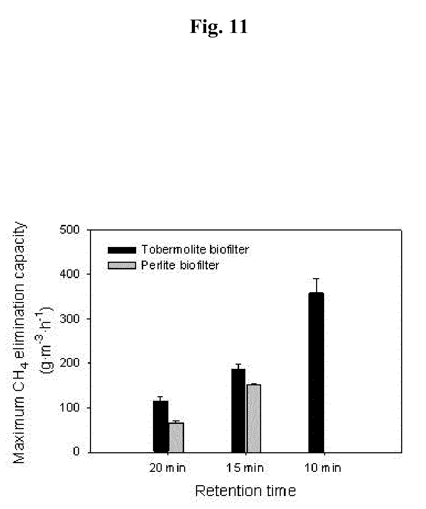

The inventors of this invention introduced tobermolite with immobilized Methanotroph to the soil in order to accelerate its methane oxidation. There was no oxidation of Methane when solely soil was used, while minor oxidation of soil was shown when tobermolite was introduced to the soil(Figure 3). Also, in a comparison of efficiency for removing of methane between a tobermolite carrier and a perlite carrier, both of which had immobilized Methanotroph, tobermolite immobilized with Methanotroph was more efficient in removing methane than perlite immobilized with Methanotroph.(Figure 4 and Figure 11). Also, as a result of long-term culturing under condition of supplying methane to the tobermolite carrier and perlite carrier which had immobilized Methanotroph, biofilm formed on the surface of perlite that resulted in decrease of availability as a biofilter while there were no forming of biofilm on the surface of tobermolite, showing no decrease of availability as a biofilter.

As another embodiment, this invention provides a biofilter or biocover that comprise tobermolite-immobilized Methanotroph carrier. The biofilter can be constituted by solely tobermolite immobilized Methanotroph which is a necessary constituting factor that can remove methane, as the biofilter shows function of removing methane by use of Methanotroph. Further, the biofilter may comprise a packing section that can contain the above tobermolite with immobilized Methanotroph or the above tobermolite as a necessary constituting factor, and additional constituting factors such as a blower, a watering system and a drainage container to efficiently remove methane from polluted gas comprising methane using the above packing section.

In this invention, the method of immobilizing the Methanotroph to the carrier is not particularly limited. However, preferable method is applying an inoculum that comprises methanotroph to the dried tobermolite and afterward drying the tobermolite. More preferable method of immobilizing the Methanotroph to the carrier comprises steps of obtaining suspension by suspending inoculums(Earthworms Cast, landfill cover soil, wetland soil, etc.) in water, gravitationally setting the suspension by maintaining a static condition for a certain period of time, obtaining supernatant, applying the supernatant to the dry carrier and drying it.

The biofilter of this invention can comprise a packing section with tobermolite carrier having immobilized Methanotroph for purpose of removing the methane contained in the gas that is injected to the biofilter.

Tobermolite carrier comprised in the packing section can be constituted of solely tobermolite or mixture of tobermolite with other carrier, although not limited to description thereof.

Also, for purpose of enhancing the methane removing effect of the biofilter, elements such as blower, watering system and drainage container can be added to the biofilter.

To elaborate, the blower obtains methane by way of suction from its occurring location and provides it to the biofilter; circulation system comprises circulation pump and watering device intended to maintain 70% or more water content in the carrier by providing water for purpose of sustaining activity of Methanotrophand preventing dryness of the carrier during operation of the biofilter; drainage container can preserve extra water content unused in the packing section and provide water to the watering system, providing various nutrients required for growth of Methanotroph to the water content. Constitution and additional constituents of devices is not particularly limited, if biofilter of this invention shows methane removing effect.

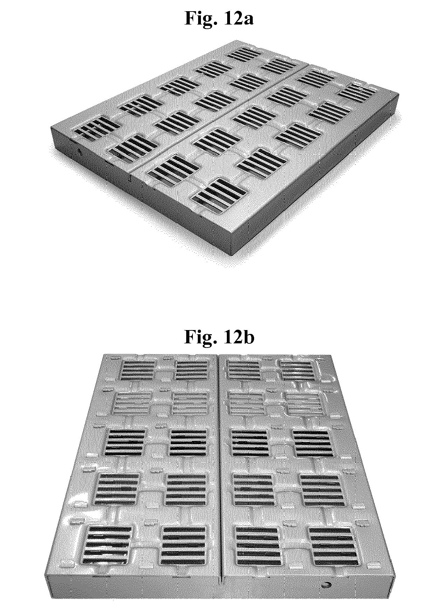

Further, biocover of this invention comprising the biofilter can be used practically to remove methane from the location of its occurrence. To illustrate, biocover of this invention can be installed to the ventilation opening or vent hole of a small scale landfill that would be economically inefficient to install a methane collection facility, or a landfill that has reached full capacity considerable time ago and discharges methane that is too low in concentration to be used as energy, for the purpose of removing the methane discharged from the landfill(Figure 12a and 12b). Figure 12 depicts an example of biocover that comprises tobermolite biofilter of this invention. (a) shows a biocover without the biofilter tobermolite, and (b) shows a biocover attached with the tobermolite biofilter. As observed in Figure 12, biocover of this invention can be a shape of a lid that could be attached to the ventilation opening or vent hole of a landfill, and midsection of the lid may be designed in a way that tobermolite biofilter of this invention can be inserted and attached in form of a unit. In this regard, the tobermolite biofilter can become a carrier(tobermolite or mixture ofother carrier and tobermolite) comprising Methanotroph, although not limited to the description thereof.

A blower, watering system, drainage container and such, which are part of elaborated biofilter system, can be added to the biocover to enhance methane removing effect of tobermolite biofilter, although constituting factors thereof are not particularly limited.

As another embodiment, this invention provides the method to remove methane using the biofilter or biocover. Specifically, methane removing method of this invention comprises a step of installing the biofilter or biocover to the location where methane is discharged. In this respect, the location where methane exist can be a location such as a landfill, although not particularly limited.

As another embodiment, this invention provides media for selectively culturing Methanotroph.

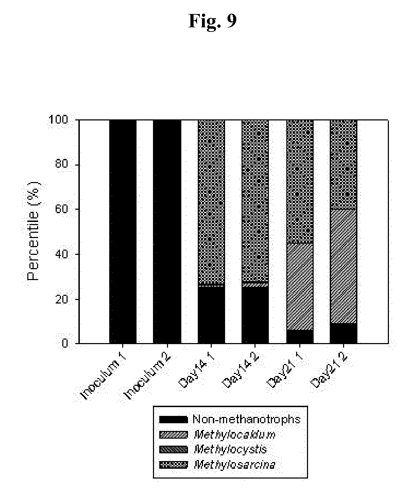

The inventors of this invention immobilized a mixed bacteria which comprises Methanotroph and other bacteria to the tobermolite or perlite. After culturing the mixed bacteria by providing it with methane, it was observed that the Methanotroph in the total bacteria group was 7.7% when the immobilized bacteria was cultured in the perlite, but the Methanotroph in the total bacteria group was 93.5% when the immobilized bacteria was cultured in the tobermolite. This increased in proportion with the length of the cultured period (Figure9).

As another embodiment, this invention uses the media to provide a method for selectively culturing Methanotroph. In detail, the method of selectively culturing Methanotroph of this invention comprises the steps to inoculate the Methanotroph to a culture media comprising tobermolite and culturing the Methanotroph by providing it with methane.

### [Advantageous Effects]

This invention can culture Methanotroph selectively and with high proliferation, while increasing efficiency of removing methane and removable amount of methane. Therefore this invention can be widely used in development of biofilter for removing stench or methane.

### [Description of Drawings]

Figure 1. Schematic diagram of biofilters.

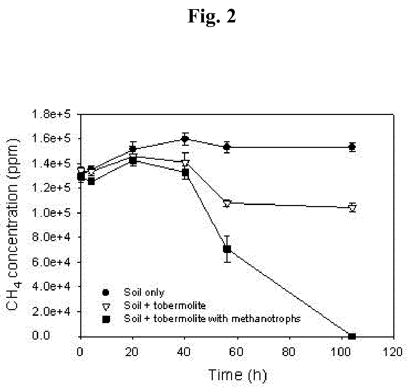

Figure 2. Potential of tobermolite as a carrier of Methanotrophs. Methanotrophs immobilized in tobermolite was introduced into soil for enhancing the soil methane-oxidation.
Figure 3. Comparison of Methanotrophic performances of short-term operated tobermolite (a) and perlite (b) biofilters.
Figure 4. Comparison of methane elimination capacities of short-term operated tobermolite and perlite biofilters. The data of the day 0 were excluded.
Figure 5. Comparison of bacterial communities of short-term operated tobermolite (a) and perlite (b) biofilters. Arrows indicate methanotrophs. Genera with assigned read numbers ≤ 0.2% of the sequencing effort were excluded. Only genera observed in either library are shown.
Figure 6. Comparison of Methanotroph proportions in communities of short-term operated tobermolite (a) and perlite (b) biofilters.
Figure 7. Methanotrophic performance of tobermolite biofilter for studying temporal variation of community structure. Inlet and outlet methane concentrations (b) and removal efficiency (b).
Figure 8. Temporal variation of microbial community in tobermolite biofilter (n=2). (a), Inoculum; (b), day 14; and (c), day 21. Arrows indicate methanotrophs. Genera with assigned read numbers ≤ 0.2% of the sequencing effort were excluded. Only genera observed in either library are shown.
Figure 9. Temporal variation of Methanotroph proportions in tobermolite biofilter.
Figure 10. Methanotrophic performances of long-term operated tobermolite (a) and perlite (b) biofilters.
Figure 11. Methane elimination capacities of long-term operated tobermolite (a) and perlite (b) biofilters.
Figure 12. An example of biocover that comprises tobermolite biofilter of this invention. (a) shows a biocover without the biofilter tobermolite, and (b) shows a biocover attached with the tobermolite biofilter.
Figure 13. Concentration of methane extracted from the inlet and the outlet of biocover is shown relative to time.
Figure 14. Gas profile according to progression of operationof biocover.

### [Mode for Invention]

A better understanding of the present invention may be obtained through the following examples which are set forth to illustrate, but are not to be construed as limiting, the present invention.

### EXAMPLE 1: Materials and Method

### EXAMPLE 1-1: Filter bed materials

Tobermolite and perlite were used as inorganic filter bed materials for this example. Their diameters were 4 to 8 mm. After they were thoroughly washed by tap water, their water holding capacities, pH (1:5), bulk densities, particle densities, porosities, surface areas and intrusion volumes were determined. Surface areas and intrusion volumes were measured using WIN9400 Series mercury intrusion porosimetry (Micromeritics, Norcross, USA). The results are listed in Table 1. Media experiments were used to confirm that these materials were unable to absorb methane (data not shown).

### EXAMPLE 1-2: Comparison between short-term operated tobermolite and perlite biofilters

Laboratory-scale biofilters, made of cylindrical acrylic resin, were used in this example. Each biofilter consisted of three parts: a packing section, watering system and medium container. The height and inner diameter of the packing section were 100 and 8 cm (approximately 5 L), respectively. The biofilters contained a perforated plate at the bottom in order to allow gases to be evenly spread. The filter materials prepared above were packed into the packing column and the working volume was 5 L. The watering system, which was 20 cm in height with an 8 cm inner diameter, contained a water inlet port at the top and a perforated plate 5 cm away from the inlet. The medium container, which was 20 cm in height with a 15 cm inner diameter, was approximately 3.5 L. These were assembled (watering system-packing section-medium container) by rubber packing and six bolts/nuts. After setting up, the gas tightness of the reactor was verified with water and compressed air. Inoculum was prepared from packing materials (perlite and granular activated carbon, 10:1 w:w) of a lab-scale Methanotrophic biofilter (Kim, T.G., et al., Microbial community analysis of a methane-oxidizing biofilm using ribosomal tag pyrosequencing. J. Microbiol. Biotechn., in press., 2012) which had been overwintered without methane supply. The packing materials were thoroughly mixed with NMS medium (1:10 w:w). NMS contains 1 g of MgSO4·7H2O, 0.295 g of CaCl2·2H2O, 1 g of KNO3, 0.26 g of KH2PO4 and 0.41 g of Na2HPO4·2H2O. CuSO4 was added to reach a final concentration of 30 µM. After mixing, a static condition was maintained for 30 min. The supernatant was used as an inoculum for the biofilters. The inoculum (2.5 L) was added into the medium containers and circulated 4 times per day for 7 days to allow cell attachment to the packing materials.

A synthetic gas composed of methane and compressed air was humidified by passing through 50 cm long humidifiers. The synthetic gas (air and CH₄) was continuously introduced into the bottom of the biofilters at a flow rate of 250 mL/min (a space velocity of 3/hr). Gas flows were controlled with commercial flow-meters (Dwyer Inc., Michigan city, USA and Kofloc Inc., Kyoto, Japan). Methane inlet concentrations were maintained at 20,000 to 100,000 ppm. The biofilters were operated at 20 ± 5°C for 23 days, in a modified NMS medium. The modified medium contains 1 g of MgSO₄·7H₂O, 0.295 g of CaCl₂·2H₂O, 10 g of KNO₃, 2.6 g of KH₂PO₄ and 4.1 g of Na₂HPO₄·2H₂O per 1 L. CuSO₄ was added to achieve a final concentration of 30 µM. During the operation, medium was circulated 4 times per day and replaced every week.

### EXAMPLE 1-3: Gas analysis

Gases were sampled from inlets and outlets of the biofilters using 0.5 mL gastight syringes (Hamilton, Reno, USA) equipped with teflon minnert fittings. Methane concentrations were monitored using gas chromatography (GC, 6850N, Agilent Technologies, Santa Clara, USA), equipped with a flame ionization detector and a wax column (30 m×0.32 mm×0.25 µm, Supelco, Bellefonte, USA). N₂ was used as a carrier gas. The temperatures of the oven, injector and detector were fixed at 100, 250 and 250°C, respectively.

### EXAMPLE 1-4: DNA extraction

Filter bed materials were collected from the biofilters at the end of the experimental period. Cells were detached from the filter bed materials by three different ultrasonic conditions using a Q500 ultrasonic processor (Qsonica, Newton, USA). A 10 g sample was added to a sterile conical tube (50 mL) containing 20 ml of sterile saline solution (0.9%). A tube was placed on bucket of ice to prevent thermal effect of ultrasound and the horn tip (6 mm diameter) of the processor was immersed about 1 cm in the solution. Ultrasonic was applied for 5 min. The actual power delivered to the suspension was 0, 10 or 50 W at a fixed frequency of 20 kHz. Then, it was agitated at 250 rpm for 30 min. A 1.5 mL suspension was transferred to a 1.5 mL microtube, and centrifuged at 16,000 x g for 10 min. The supernatant was discarded from the tube. DNA was extracted using the NucleoSpin Soil kit (Macherey-Nagel GmbH, Düren, Germany), by a modified method of applying BeadBeater-8 system (BioSpec, Bartlesville, USA) at 5,000 rpm for 30 secs to the sample. DNA was eluted using 100 µL of the elution buffer and stored at 20°C prior to use. DNA was quantified using an ASP-2680 spectrophotometer (ACTGene, Piscataway, USA).

### EXAMPLE 1-5: Polymerase chain reaction (PCR) and pyrosequencing

A total of 6 different pyrosequencing libraries were produced (2 biofilters x 3 ultrasonic treatments). For PCR, the primer set 340F and 805R was used to amplify the 340-805 region of the 16S rRNA gene (positions based on Escherichia coli) containing the V3 and V4.
340F: 5'-TCCTACGGGAGGCAGCAG-3'(Sequence ID No. 1)
805R: 5'-GACTACHVGGGTATCTAATCC-3'(Sequence ID No. 2)

Composite primer sets for multiplex pyrosequencing(Sequence ID No. 3 and 4) were made based on the 340F-805R sequence:
forward primer 
5’-CCATCTCATCCCTGCGTGTCTCCGACTCAGNNNNNNNNNNTCCTACGGG
AGGCAGCAG-3’(Sequence ID No. 3)
reverse primer

5’-CCTATCCCCTGTGTGCCTTGGCAGTC-TCAG-GACTACHVGGGTATCTAA
TCC-3’ (Sequence ID No. 4)

For each DNA sample, four independent PCR mixtures were prepared in parallel to avoid PCR bias. The 50-µL mixtures contained 5 µL of 10X PCR buffer (Genenmed, Seoul, Korea), 10 µg of bovine serum albumin, 2 µL of each composite primer (10 µM), 1 U of Ace Taq polymerase (Genenmed), 4 µL of 2.5 mM dNTPs and approximately 150 ng of template DNA. The reactions were performed(95°C for 3 min, 25 cycles (94°C for 45 secs, 50°C for 45 secs and 72°C for 45 secs) and at 72°C for 5 mins) in a 2700 GeneAmp PCR system (Applied Biosystems, Foster, USA).

Four PCR products were combined and the PCR products were subjected to electrophoresis on 2% low melting agarose gel. The expected size of the PCR products was about 530 bp. The agarose gel containing the PCR products were excised. After, DNA from the agarose gel was purified using the QIAquick Gel extraction kit (Qiagen, Valencia, USA), as described in the manufacturer's instructions.

The concentration of the purified DNA was measured using an ASP-2680 spectrophotometer (ACTgene). The same amount of purified DNA was combined in a single tube to produce pyrosequencing libraries, and sent to Macrogen Incorporation (Seoul, Korea) to be run on a Genome Sequencer 454 FLX Titanium system (Roche Diagnostics, Mannheim, Germany).

### EXAMPLE 1-6: Analysis of pyrosequencing results

High-quality sequences were obtained using several steps: Trimming primers, removing low quality DNA sequences (length < 350 nt; average quality score < 25; and with an ambiguity) using the quality trimming tool of the RDP pyrosequencing pipeline (Cole et al., 2009), and excluding chimeras with Black Box Chimera Check software (Gontcharova et al., 2010). Pyrosequencing reads were taxonomically assigned using the RDP-classifier of the pipeline with bootstrap values more than 80%. The RDP classifier provides rapid and independent taxonomic classifications from domain to genus. The classification result was analyzed using MEGAN software version 4.50 (Huson et al., 2007). Reads assigned to Methanotrophic genera (Iguchi et al., 2011; Semrau et al., 2010) were assumed to be Methanotrophs, and their proportions in the pyrosequencing libraries were calculated.

### EXAMPLE 1-7: Temporal change of bacterial community in tobermolite biofilter

A lab-scale biofilter with tobermolite was operated for 20 days, with the same conditions as previously described above. Inoculum was prepared from the bed material of the perlite biofilter (the section 2.2). The perlite was mixed with NMS medium (1:10 w:w). After mixing, a static condition was maintained for 30 min. The supernatant (2.5 L) was added into the medium container.

DNA was extracted from tobermolite on days 14 and 21, as well as from the inoculum. Cells were detached from tobermolite and DNA was extracted by the method described above. The actual power of 10 W was applied in this experiment. A 1.5-mL aliquot of the inoculum was transferred to a 1.5-mL microtube, and centrifuged at 16,000 x g for 10 min. The supernatant was discarded. DNA was extracted as described above. There were two replicates per each sample. Pyroseqeuncing was performed and analyzed as described above.

### EXAMPLE 1-8: Long term operation of biofilters

Two tobermolite and perlite biofilters were set up as described in the Example 1-2. Inoculum was prepared from the bed material of the perlite biofilter as previously described in the Example 1-7. The synthetic gas (air and CH₄) was continuously introduced into the bottom of the biofilters at flow rates of 250 to 500 mL/min (a space velocity of 3 to 6/hr). Methane inlet concentrations were maintained at 20,000 to 140,000 ppm. The biofilters were operated at 20 ± 5°C for 174 and 204 days for the tobermolite and perlite biofilters, respectively, with the modified NMS medium. During the operation, medium was circulated 4 times per day and replaced every week.

### EXAMPLE 1-9: Potential of tobermolite as a carrier of Methanotrophs

Fresh tobermolite was thoroughly washed with tap water. Tobermolite was collected from the long-term operated biofilter on day 160. They were allowed to dry for a week under a room condition. Soil was collected from a landfill cover (Gapyeong-gun, Kyungido, Korea). Soil was dried and sieved through 2 mm mesh. 80 grams of soil and 20 grams of tobermolite were thoroughly mixed, adjusted to a soil moisture content of 20%, and placed into 600 mL serum bottles. Serum bottles were sealed with butyl-rubber stoppers and methane was injected to reach a final concentration of 100,000 ppm. They were incubated at a room temperature. Methane concentrations in the headspace were monitored over time using gas chromatography. There were three treatments: i) soil only; ii) soil + tobermolite; and (iii) soil + tobermolite with Methanotrophs. There were five replicates for each sample.

### EXAMPLE 2: Results and discussion

### EXAMPLE 2-1: Potential of tobermolite as a carrier of Methanotrophs

Methanotrophs immobilized in tobermolite was introduced into soil to enhance the methane oxidation of the soil. No apparent methane oxidation was observed when solely soil was used and little oxidation was shown in the usage of soil with tobermolite (Figure. 3). Methane was completely consumed within 105 hours in soil when Methanotrophs immobilized in tobermolite was applied. The results indicate promising potential of tobermolite for formulating Methanotrophs to apply Methanotrophs into soil environments and engineering systems.

### EXAMPLE 2-2: Comparison of Methanotrophic performances of short-term operated tobermolite and perlite biofilters

Two identical biofilters were set up and tobermolite and perlite were used as packing materials.

**[Table 1]**

| Physicochemical characteristics of tobermolite and perlite. | | |
|---|---|---|
| Characteristic | Tobermolite | Perlite |
| Water holding capacity (%) | 109.5±5.1 | 64.6±12.4 |
| pH | 7.0±0.1 | 6.0±0.0 |
| Bulk density (g/cm³) | 0.276±0.000 | 0.470±0.002 |
| Particle density (g/cm³) | 1.050±0.022 | 1.248±0.048 |
| Porosity (%) | 73.7±0.6 | 62.3±1.4 |
| Surface area (m²/g) | 122.0 | 27.0 |
| Intrusion volume (mL/g) | 0.7638 | 1.2868 |

Noticeably, surface area of tobermolite is 4 to 5 times greater than that of perlite, and is comparable to those of clay minerals such as illite and montmorillonite. Figure 3 shows the methane removal efficiencies (RE) in tobermolite and perlite biofilters. Methane REs were stabilized after 7 days in the both biofilters. Methane elimination capacities (EC) were plotted with the inlet loads (Figure 4). For data analysis, results for the first 7 days were excluded. EC was 73.7 ± 17.4 and 52.5 ± 13.3 g/m³h at loads of 133.7 ± 26.6 and 114.3 ± 26.6 g/m³h for the tobermolite and perlite biofilters, respectively. EC increased along with methane loads. Slope values, which correspond to methane REs, were calculated to be 0.54(54%) and 0.44(44%) for the tobermolite and perlite biofilters, respectively. The tobermolite biofilter was more efficient for methane removal than the perlite biofilter.

### EXAMPLE 2-3: Comparison of bacterial communities of short-term operated tobermolite and perlite biofilters

A total of 6 pyrosequencing libraries with average read lengths of 416-424 bp were obtained after the filtering steps (Table 2).

**[Table 2]**

| The sequence numbers and average read lengths of pyrosequencing libraries. | | | |
|---|---|---|---|
| | Sample | N | length |
| The first run of biofilter | Tobermolite (0 W) | 14,051 | 423.8 |
| | Tobermolite (10 W) | 6,970 | 423.9 |
| | Tobermolite (50 W) | 8,943 | 424.2 |
| | Perlite (0 W) | 1,793 | 420.0 |
| | Perlite (10 W) | 1,242 | 421.2 |
| | Perlite (50 W) | 1,377 | 416.2 |
| The second run of biofilter | Inoculum-1 | 310 | 418.8 |
| | Inoculum-2 | 477 | 418.2 |
| | Day 14-1 | 599 | 423.9 |
| | Day 14-2 | 796 | 423.8 |
| | Day 21-1 | 696 | 425.5 |
| | Day 21-2 | 136 | 424.7 |

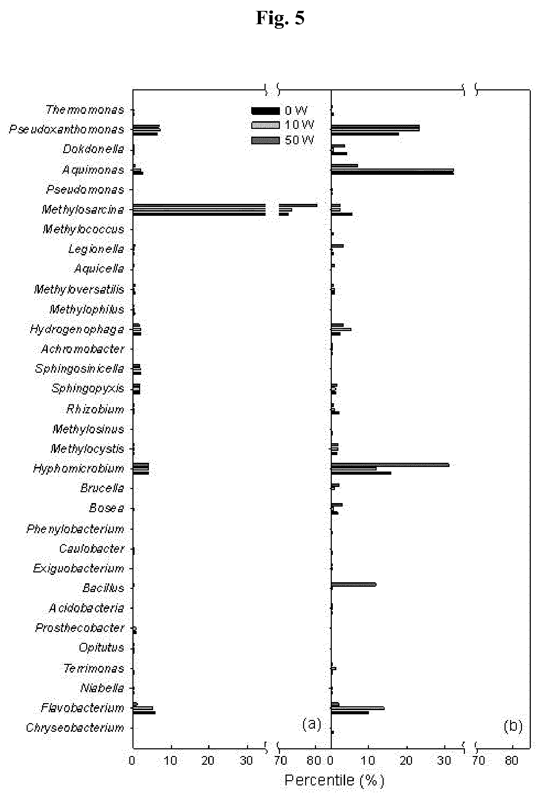

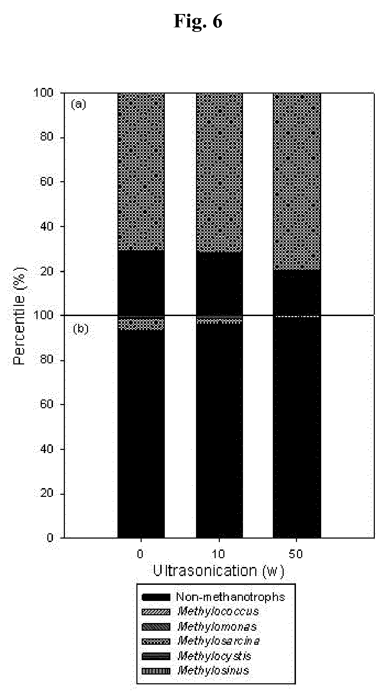

All sequences were individually identified at the genus level (Figure 5). Methylosarcina was most abundant in the tobermolite biofilter, making up to 82.6% (50 W), followed by Pseudoxanthomonas (7%), Flavobacterium (4%) and Hyphomicrobium (4%). In contrast, Aquimonas (23%), Pseudoxanthomonas (21%), Hyphomicrobium (20%), Flavobacterium (9%) and Bacillus (4%) were abundant and Methanotrophs constituted up to 7.7% (0 W) in the perlite biofilter. Methylosarcina was found to be a sole Methanotroph in the tobermolite biofilter, while there were four Methanotrophic genera such as Methylococcus (0.17% of the community), Methylocystis (1.63%), Methylosarcina (3.4%) and Methylosinus (0.13%) in the perlite biofilter. Methylosarcina was most abundant in the both biofilters. Methanotrophs are generally grouped into types I and II by physiological characteristics and phylogenetic affiliation (Semrau et al., 2010). All observed Methanotrophic genera belong type I group, except for Methylocysitis. The both biofilters were predominated by type I members. It has been observed that type II Methanotrophs are more abundant than type I members in Methanotrophic biofilters and biofilm. Ultrasound has been commonly used to detach cells and biofilms from substrata. As the ultrasonic input power increased, Methanotrophic proportion increased from 72.0% to 82.6% in tobermolite, while decreased from 7.7% to 4.1% in perlite (Figure 6). The results suggested that Methanotrophs were more tightly inoculated to tobermolite than to perlite.

### EXAMPLE 2-4 Temporal variation of community composition in tobermolite biofilter

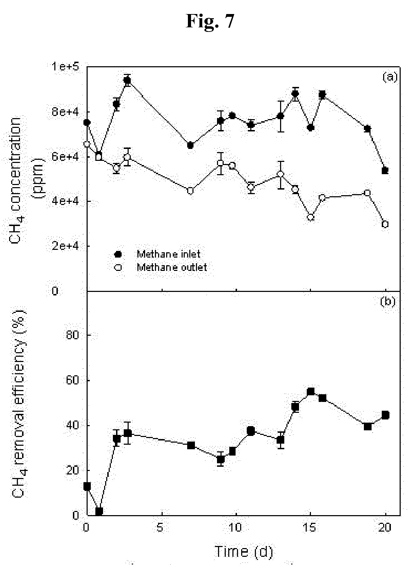

To verify tobermolite as a selective bed material for Methanotrophs, the biofilter experiment with tobermolite was repeated with the same conditions. The used inoculum was obtained from the perlite biofilter as mentioned in the Example 2-7. RE and EC results are shown in Figure 7, were similar with those of the short-term operated biofilter. Methane RE was stabilized after 3 days. For data analysis, results for the first 3 days were excluded. EC was 62.1 ± 20.7 g/m³h at a methane load of 144.1 ± 31.5 g/m³h.

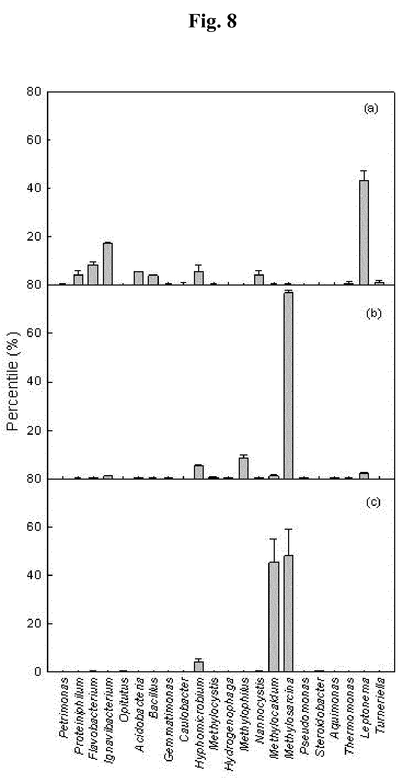

A total of 6 pyrosequencing libraries with average read lengths of 418-426 bp were obtained after the filtering steps (Table 2). Sequences were individually identified at the genus level (Figure 8). Methanotrophs including Methylocystis, Methylosarcina and Methylocaldum were observed in the biofilter. Methanotrophs dramatically increased over time (Figure 9). Non-Methanotrophs such as Leptonema, Ignavibacterium, Flavobacterium, Hyphomicrobium, Acidobacteria, Proteiniphilum, Nannocystis and Bacillus were abundant in the inoculum, while Methanotrophs were present in less than 1%. Methanotrophs made up 78.6% of the community on day 14, with Methylosarcina (76.8% of the community), Methylocaldum (1.2%) and Methylocystis (0.7%). Methylotrophs (single carbon degrader) such as Methylophilus (8.8%) and Hyphomicrobium (5.4%) were observed. Methanotrophs comprised 93.5% at day 21, with Methylosarcina (48.1% of the community) and Methylocaldum (45.5%). To the best of our knowledge, this is the highest degree of selectivity(over 93% of total bacteria) for growth of Methanotrophs in Methanotrophic biofilter systems(over 93% of bacteria). The results demonstrate that tobermolite is a carrier for Methanotrophs that is excellent in selective growth, showing selective Methanotroph growth of up to 93% of total bacteria in the tobermolite carrier.

### EXAMPLE 2-5: Long term operation of tobermolite and perlite biofilters

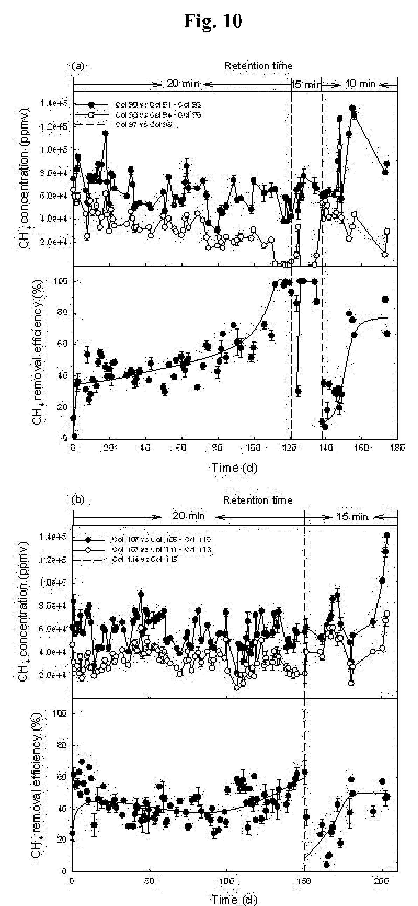

Figure 10 shows the removal efficiencies (RE) of methane in the long-term operated tobermolite and perlite biofilters. RE increased rapidly for the first few days in the both biofilters.

RE increased with time for the first 120 days in the tobermolite biofilter. Under 20 mins of detention time, methane RE reached close to 100%. Afterwards, methane load was stepwise raised by way of reducing the gas detention time. Methane RE was low as the gas detention time was reduced to 15 mins. However, after few days the methane RE increased to almost 100%. When the gas detention time was reduced to 10 mins, methane RE increased to approximately 80%. Maximum level of methane RE in tobermolite biofilter was 357.8g/m³h(value corresponding to 8,592g/m³d). Further, RE maintained at 40 to 50% for the first 150 days in the perlite biofilter under gas detention time of 20 mins. On the day 150 when RE value reached 60%, gas detention was reduced to 15 mins resulting in methane RE value of 50%. Maximum amount of removed Methane was 66.3 ± 3.4 and 151.4 ± 2.0g/m³h under 20 and 15 mins detention time, respectively. Maximum level of methane RE in perlite biofilter was 151.4g/m³h(value corresponding to 3,633g/m³d). Thus, it was proven methane RE is higher in long term operation of biofilter when using tobermolite, compared to perlite biofilter.

**[Table 3]**

| Methane elimination capacities of filter bed materials. | | | | | |
|---|---|---|---|---|---|
| Filter bed material | Operating condition | Space velocity | Maximum elimination capacity (g/m³d) | Efficiency | Reference |
| Tobermolite | Lab scale | 6 | 8,587 | 74% | This invention |
| Perlite | Lab scale | 4 | 3,634 | 51% | This invention |
| Perlite+activated carbon | Lab scale | 3 | 2,544 | 83% | Unpublished |
| Compost | Lab scale | 4.7 | 1,488 | N/A | Ref. 1 |
| Compost+peat+ wood Fibers | Lab scale | 4.7 | 960 | N/A | Ref. 1 |
| Multi-layers | Lab scale | 4.7 | 720 | N/A | Ref. 1 |
| Inorganic gavel+stone | Lab scale | 14.4 | 1,440 | 66% | Ref. 2 |
| composted pine bark+perlite | Lab scale | 0.3 | 137 | 60% | Ref. 3 |
| inorganic material | Lab scale | 14 | 701 | 41% | Ref. 4 |
| mature compost | Lab scale | 14 | 300 | 19% | Ref. 4 |
| inorganic stone | Lab scale | 14 | 1,073 | 60% | Ref. 5 |
| Porous clay | Lab scale | 14.6 | 408 | 19% | Ref. 6 |
| Rock material (5 mm) | Lab scale | 14.6 | 912 | 42% | Ref. 6 |
| Rock material (2 mm) | Lab scale | 14.6 | 1,200 | 56% | Ref. 6 |
| Soil+earthworm cast | Lab scale | 11.8 | 6,818 | 68% | Ref. 7 |
| Compost | Pilot scale | 6.3 | 960 | N/A | Ref. 1 |
| Compost+peat+ wood fibers | Pilot scale | 6.3 | 480 | N/A | Ref. 1 |
| Compost | Pilot scale | <0.516 | 672 | 100% | Ref. 8 |
| Wood chips+compost | Pilot scale | <0.516 | 576 | 100% | Ref. 8 |
| Perlite+compost | Pilot scale | 0.8-8.6 | 216 | N/A | Ref. 9 |

| | | | | | |
|---|---|---|---|---|---|
| Ref. 1: Streese, J., et al., Waste Manage., 23:573-580, 2003 Ref. 2: Josiane, N., et al., The Canadian Journal of Chemical Engineering, 87:136-142, 2009 Ref. 3: Plessis, C.A.d., et al., Fuel, 82:1359-1365, 2003 Ref. 4: Nikiema, J., et al., Chem. Eng. J., 113:111-117, 2005 Ref. 5: Nikiema, J., et al., Can. J. Civ. Eng., 37:335-345, 2010 Ref. 6: Nikiema, J., et al., International Journal of Chemical Engineering, 2010:1-8, 2010 Ref. 7: Park, S., et al., Water, Air, & Soil Pollution, 196:19-27, 2009 Ref. 8: Haubrichs, R., et al., Waste Manage., 26:408-416, 2006 Ref. 9: Melse, R.W, et al., Environ. Sci. Tech., 39:5460-5468, 2005 | | | | | |

In comparison of tobermolite biofilter with biofilters that uses various types of carriers that are publicly known, tobermolite biofilter shows the greatest methane elimination capacities (EC), compared with the reported values. The results demonstrated that methane removal efficiencies(RE)is very high when tobermolite is used as a carrier. Clogging is caused mostly by overgrowth of microbes and results in the bed pressure drop which in turn increases the operating costs and decreases the efficiency. On day 15, biofilms were apparently visible in the column for the perlite biofilter.

Due to significant clogging and channeling problems in the perlite biofilter, the packing material was washed out on days 35 and 110 to remove the excessive biomass. It was observed that there were no apparent clogging and channeling problems in the tobermolite biofilter for the long-term operation. This indicated that tobermolite can be an excellent filter bed material for methane removal in terms of operation management.

The result of the Example 2 shows that selective growth of microorganism intended to be inoculated to a filter carrier provides many advantages such as easy usage and management.

In this invention, tobermolite and perlite was used as a filter carrier for removing methane. The tobermolite biofilter showed better methane RE compared to perlite biofilter. Pyrosequencing result demonstrated that Methanotroph was a dominant species in tobermolite, while remaining as a fraction in perlite. By way of repeated biofilter experiments, it was verified that Methanotroph was 93.5% in tobermolite.

Observation made through progression of time demonstrated selective growth of Methanotroph in tobermolite. Therefore, tobermolite provides an ideal habitat for selective growth of Methanotroph. The result shows potential of tobermolite as a promising biofilter carrier in aspect of effect and habitat of Methanotroph.

### Example 3: Biocover System

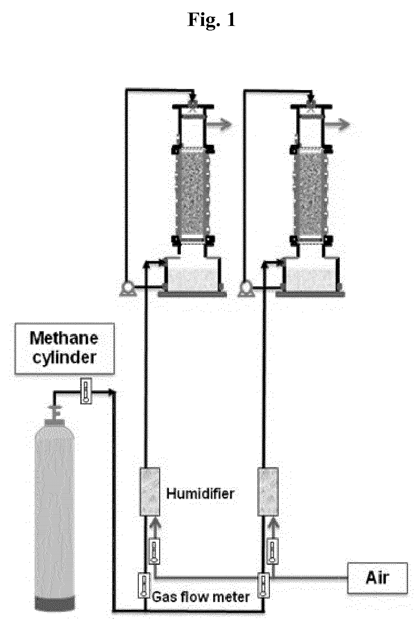

Lab scale biocover was produced to investigate the methane removing feature of a functional biocover that uses tobermolite as a main packing material. Lab scale biocover was produced using acrylic column. Detailed diagram of the device is on figure 1. Biocover comprise of packing section which is 8 cm in diameter and 50cm in height, ventilation section which is 8 cm in diameter and 15 cm in height as well as gas injection section which is 8 cm in diameter and 5 cm in height. Filling material was filled into the packing section of the biofilter after mixing Ga-pyeong landfill soil, earthworm cast and tobermolite by way of 0.25:0.25:0.5(w/w/w).

The gas injection section in lower part of the biocover was filled with orchid stones (4-8 mm in diameter) to evenly provide the gas to the packing layer of the biocover. To prevent drying of the biocover, (Methane/carbon dioxide (40:60% (v/v), Seoul special gas, Korea) gas was provided to the biocover at speed of 5 mL/min after passing it through a humidifier. The ventilation section in the upper part of biocover duplicated the air condition close to the landfill soil by injecting air at a flow rate of 20 ml/min. The biocover was operated for 134 days at room temperature (30s°C). During the operation of the biocover, 300*µℓ* of samples were extracted every 1 to 2 days from sampling ports located at entrance and exit of the biocover, by using a 500*µℓ* gastight syringe. Removal rate of each gas was calculated by analyzing the concentration of each gas from the sample using GC-FID and observing the difference in concentration between gas deriving from entrance and the exit.

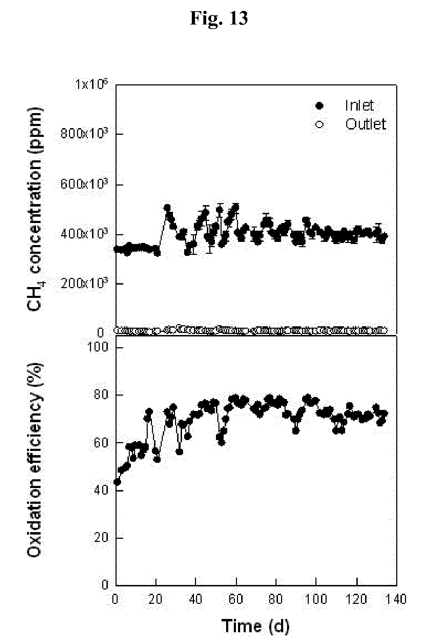

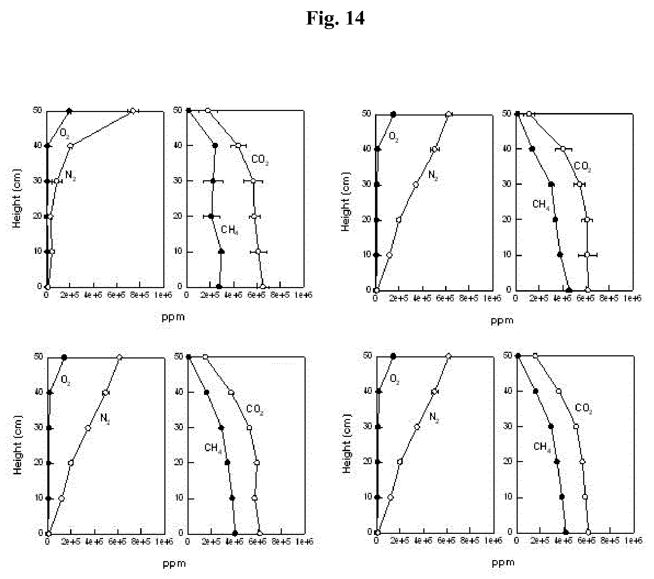

Also, in order to monitor gas profile according to depth of the biocover, concentration of O₂ and N₂, CH₄ as well as CO₂ was analyzed using GC with thermal conductivity detector(TCD)(Figure 13 and 14).

In providing a mixed gas of 40% methane and 60% CO₂(v/v) at speed of 5 ml/min to the lab scale biocover that uses tobermolite as a main packing material, concentration of methane extracted from the inlet and the outlet of biocover is shown relative to time in Figure 13. In the initial stage of operation, methane showed decomposition rate of under 60%. However, as time progressed and efficiency increased, it was stabilized(after day 30), showing an average of 73ter in methane RE. The methane EC at this time is 228.7 to 291.4 g/m²d. Also, Figure 14 shows gas profile at day 25, 52, 100 and 134 according to progression of operation. It is verified that, compared to the initial stabilizing period of day 25, oxygen and nitrogen of day 52, 100 and 134, which stably maintain removing of methane, are provided in depth and methane and carbon dioxide profile are also maintained stably. Also, it is noticed through observation that concentration of methane rapidly decreases at height 30 to 50 cm, indicating that removing of methane is most active in this section. Therefore, it is verified that methane eliminating capability of a biocover that uses tobermolite as a main packing material is stably operated and maintained at high efficiency.
## Claims

1. A carrier for use in removing methane comprising tobermolite-immobilized Methanotroph.

2. The Carrier of Claim 1, wherein said Methanotroph is selected from the group consisting of Methylocystis sp., Methylosarcina sp., Methylocaldum sp. and combination thereof

3. A Biofilter comprising the carrier of Claim 1.

4. A biocover comprising the biofilter of Claim 3.

5. A method for removing methane comprising installing biofilter of Claim 3 or biocover of claim 4 to location where methane exists.

6. The method of claim 5, wherein said location is a landfill.

7. Use of a culture media comprising tobermolite for culturing selectively Methanotroph.

8. A method for selectively culturing Methanotroph comprising:
(a) inoculating a Methanotroph to a culture media comprising tobermolite; and
(b) culturing the Methanotroph while supplying methane.

## Patentansprüche

1. Träger zur Verwendung bei der Entfernung von Methan, umfassend einen tobermoritimmobilisierten Methanotrophen.

2. Träger nach Anspruch 1, wobei der Methanotroph ausgewählt ist aus der Gruppe bestehend aus Methylocystis sp., Methylosarcina sp., Methylocaldum sp. und Kombinationen davon.

3. Biofilter, umfassend den Träger nach Anspruch 1.

4. Biodeckel umfassend den Biofilter nach Anspruch 3.

5. Verfahren zum Entfernen von Methan, umfassend das Installieren eines Biofilters nach Anspruch 3 oder Biodeckels nach Anspruch 4 an einem Standort, an dem Methan vorhanden ist.

6. Verfahren nach Anspruch 5, wobei der Standort eine Mülldeponie ist.

7. Verwendung eines Kulturmediums, das Tobermorit zum selektiven Kultivieren von Methanotrophen umfasst.

8. Verfahren zum selektiven Kultivieren von Methanotrophen, umfassend:
(a) Inokulieren eines Methanotrophen auf ein Kulturmedium, das Tobermorit umfasst; und
(b) Kultivieren des Methanotrophen, während Methan zugeführt wird.

## Revendications

1. Support destiné à être utilisé pour l'élimination de méthane et comprenant un méthanotrophe immobilisé à la tobermorite.

2. Support selon la revendication 1, dans lequel ledit méthanotrophe est choisi dans le groupe constitué de Methylocystis sp., Methylosarcina sp., Methylocaldum sp. et leur mélange.

3. Filtre organique comprenant le support selon la revendication 1.

4. Couverture organique comprenant le filtre organique selon la revendication 3.

5. Méthode d'élimination du méthane comprenant l'installation d'un filtre organique selon la revendication 3 ou d'une couverture organique selon la revendication 4 dans un site où du méthane est présent.

6. Méthode selon la revendication 5, dans laquelle ledit site est une décharge.

7. Utilisation d'un milieu de culture comprenant de la tobermorite pour la culture sélective d'un méthanotrophe.

8. Méthode de culture sélective d'un méthanotrophe comprenant :
(a) l'inoculation d'un méthanotrophe à un milieu de culture comprenant de la tobermorite ; et
(b) la culture du méthanotrophe pendant la fourniture de méthane.
